# EUROPÄISCHE PATENTSCHRIFT

(11) **EP 3 018 102 B1**
(45) Veröffentlichungstag und Bekanntmachung des Hinweises auf die Patenterteilung: **12.04.2023**
(21) Anmeldenummer: 14002051.2
(22) Anmeldetag: 13.06.2014
(51) Int. Cl.: C02F 1/42

(54) **Vorrichtung zur Überwachung der Gesamtchlormessung einer Wasseraufbereitungsanlage**
Dispositif de contrôle de la mesure de chlore total d'une station d'épuration
Device for monitoring the total chlorine measurement of a water treatment plant

(30) Priorität: 13.07.2013 DE 102013011752
(43) Veröffentlichungstag der Anmeldung: 11.05.2016
(73) Patentinhaber: Vivonic GmbH, 63877 Sailauf (DE)
(72) Erfinder: Völker, Manfred, 63825 Blankenbach (DE)
(74) Vertreter: Herrmann, Uwe

(56) Entgegenhaltungen:
- EP-A1- 2 497 750
- EP-A1- 2 583 950
- WO-A1-2007/096577
- DE-A1- 4 312 600
- DE-A1-102005 049 169
- US-A1- 2001 004 962
- US-B1- 6 174 419

## Beschreibung

Die Erfindung betrifft ein Fluidsystem zur Qualitäts-, Funktionsüberwachung und/oder Steuerung von physikalisch und chemisch wirkenden Filterstufen einer Wasservorbehandlung für den Betrieb einer Umkehrosmose-, oder einer anderen Wasseraufbereitungs- bzw. Wasserüberwachungsanlage.

Die WO 2007/096577 A1 offenbart ein Verfahren zur Funktionsüberwachung einer Chlorsensoreinrichtung. Die EP 2 583 950 A1 offenbart ein Verfahren und eine Anordnung zur Wasserbehandlungssteuerung.

Nachteil von Filterstrecken ist die nicht oder nur aufwändig durchzuführende Ferndiagnose von Chlor und Härte und die Überwachung des Verschmutzungsgrades von mechanischen Filtern.

Außerdem ist es aus Sicherheitsgründen, insbesondere bei Dialysewasseraufbereitungen, erforderlich, täglich eine aufwändige manuelle Dokumentation der Wasserhärte und/oder des Chlorgehaltes durchzuführen, insbesondere um den Nachweis zu führen, dass das toxische Chlor aus der Flüssigkeit durch die eingesetzten Filter entfernt wurde.

Vorhandene Chlorsensoren zur Onlinemessung werden häufig nicht regelmäßig gechlort oder können bei Abwesenheit von Chlor in der Flüssigkeit keine zuverlässigen Messergebnisse liefern.

Um schwer lösbare Salze z.B. Kalzium und/oder Magnesium aus dem Wasser zu entfernen, werden häufig Enthärter eingesetzt.

Bei Verwendung von Enthärtern mit sauren Kationentauscherharzen sind diese mittels Natriumchloridsolelösung regelmäßig zu regenerieren.

Diese Regeneration wird in der Regel mit Natriumchloridlösung durchgeführt, die in einem sogenannten Solebehälter, in dem Salz in einer vorherbestimmten Flüssigkeitsmenge aufgelöst wird, bereitgestellt ist.

Ein Ausfall der Regenerierung z.B wegen fehlender Natriumchloridsolelösung kann zu gravierenden Verkalkungen der nachfolgenden Anlagen führen.

Darüber hinaus neigen Enthärter wegen der relativ großen Harzvolumen zu mikrobiellem Wachstum mit nachfolgender Kontamination der durchfließenden Flüssigkeit.

Problematisch sind Filterverblockungen, weil der daraus resultierende Austausch von Filtermaterial normalerweise mit einer Betriebsunterbrechung einhergeht.

Ziel der Erfindung ist die Entwicklung einer Aktor-Sensorsteuerung, die es dem Anwender ermöglicht, die Funktionalität einer Anlage per online-Zugriff zu bewerten und auf dieser Basis eine Ferndiagnose über den aktuellen Betriebszustand zu erhalten.

Die Erfindung wird durch die Gegenstände mit den Merkmalen der unabhängigen Ansprüche definiert. Vorteilhafte Weiterbildungen der Erfindung sind Gegenstand der abhängigen Ansprüche.

Um den normativen und/oder hausinternen Anforderungen zu genügen, können mit der automatischen Registrierung gleichzeitig die erforderlichen Dokumentationsnachweise durch die angeschlossene EDV erbracht werden.

Durch die angestrebte anlagenspezifische Auswertung durch Analyse und Visualisierung der Betriebsparameter wird es möglich, eine azyklische Verteilung der Service-Einsätze und damit eine Verringerung der Servicezahl zu erreichen.

Auf dieser Basis ist ein ökonomisches und ökologischeres Vorgehen möglich, da somit der Einsatz von geschultem Personal vor Ort besser koordiniert und verschleißbedingte Ausfälle gezielt und präventiv vermieden werden können.

Um die vorgenannten Nachteile zu vermeiden, bzw. der Zielsetzung zu entsprechen, werden nach einem Gesichtspunkt der Erfindung vor und nach den Filterstufen mittels geschalteter Ventile Teilströme zu dem entsprechenden Sensor geführt und durch elektronische Messeinrichtungen ausgewertet. Wobei diese Messeinrichtungen auch integraler Bestandteil von nachfolgenden Anlagen einer Wasseraufbereitung und/oder auch eine Leitwarte sein können und wobei ein bidirektionaler Betrieb zur Beeinflussung von Aktoren und Sensoren möglich ist.

Mit Vorteil werden mit einem elektronischen Druckaufnehmer unterschiedliche mechanische Filterstufen online auf ihren Verschmutzungsgrad durch Messung der Drücke und Ermittlung der Druckdifferenz überwacht und bei geeigneten Filtern mit entsprechender Rückspülautomatik auch ein automatisches Rückspülprogramm eingeleitet.

Erfindungsgemäß wird ein online messender Chlorsensor eingesetzt, dessen sicherheitsrelevante Funktion erfindungsgemäß überprüft wird, indem dem Sensor elektrolytisch erzeugtes Chlor regelmäßig mit bekannter Konzentration zugeführt wird. Das Messergebnis wird erfindungsgemäß elektronisch registriert und dokumentiert. Das Chlor kann aus einer vorhandenen Solelösung hergestellt werden.

Die Funktion des Enthärters, also die Filtration und Reduzierung der schwerlösbaren Calcium und Magnesiumsalze, kann durch einen ionensensitiven Calcium- und/oder Magnesiumsensor überwacht werden.

Der Füllstand des Salzsolebehälters, bzw. das Restvolumen der Salze im Solebehälter, ist einfach mittels Waage zu überwachen. Dazu wird der Solebehälter auf ein Konstruktionselement mit Wäagezelle plaziert. Da das konstruktive Untergestell unabhängig von dem verwendeten Solebehälter jederzeit einzusetzen ist, können auch bereits im Betrieb befindliche Solebehälter mit dieser Überwachungseinrichtung bestückt werden.

Es ist möglich, das Solevolumen direkt- oder als Ampellösung mit Meldungsfarbe anzuzeigen, eine Weiterleitung und Registrierung an eine Leitwarte oder einer nachfolgenden Wasseraufbereitung, die z.B. als RO Anlage ausgebildet sein kann, ist ebenfalls möglich.

Damit kann eine vom Betriebspersonal täglich durchzuführende Inspektion und Dokumentation des Salzvorrates im Solebehätter entfallen.

Ein regelmäßige leichte Chlorierung des Enthärters während der Regenerierung durch elektrolytisch aus dem Solebehälter des Enthärters erzeugtem Chlor reduziert das mikrobielle Wachstum im Enthärterharz und sorgt somit für keimfreiere Flüssigkeit.

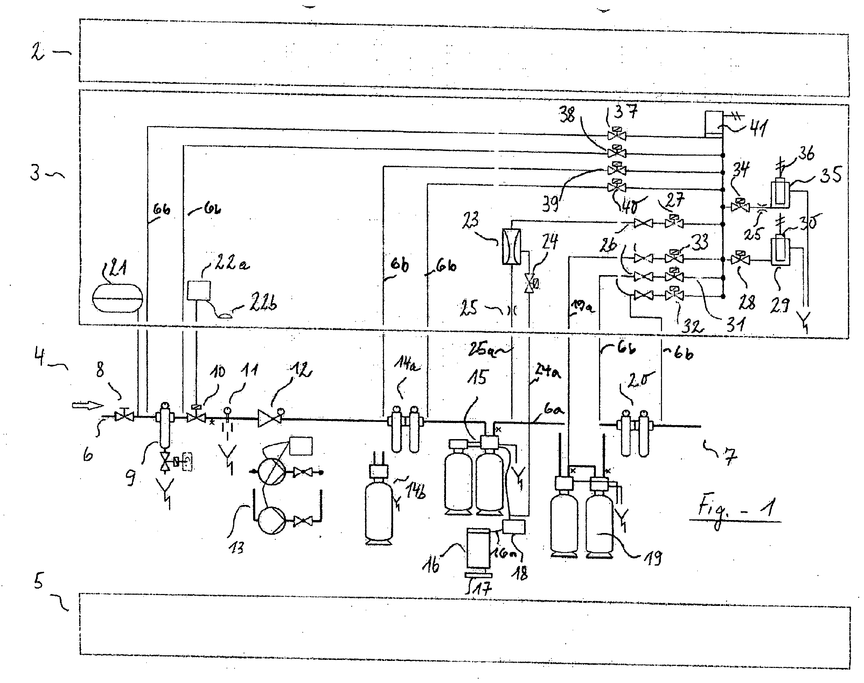

Figur 1 zeigt eine Vorfiltrationseinheit mit einer mechanisch-chemischen Filterstufe 4, eine Aktor-Sensor-Überwachungseinheit 3, eine zugehörige Auswerteelektronik 2 und eine mögliche, beispielsweise zu einer nachfolgenden Umkehrosmose gehörende Elektronik 5, wobei die Elektronik 2 auch als Leitwarten-Elektronik ausgebildet sein kann und mit Elektronik 5 kommuniziert.

Die mechanisch-chemische Filterstufe 4 ist hinsichtlich der Auswahl der angeordneten Filterstufen nur beispielhaft dargestellt, um die Funktion der erfindungsgemäßen Überwachung zu verdeutlichen.

Die beispielhafte Anordnung einer Wasserleitung 6a beginnt mit dem Wassereingang 6, einem Absperrventil 8 und einem automatisch rückspülbaren Vor-filter 9 mit Abflussventil und Drainageanschluss. Es folgt ein Sicherheitsabsperrventil 10, welches von einem Leckagemelder 22a mit Flüssigkeitsensor 22b aktiviert wird.

Weitere Komponenten können ein Rohrtrenner 11 und ein Rückflussverhinderer 12 zur Vermeidung einer Kontamination des Wassereingangs 6 sein.

Bei niedrigen Wasserzulaufdrücken besteht die Möglichkeit der Hinzunahme einer Druckerhöhungseinheit 13. Eine mögliche weitere Filterstufe 14 kann sowohl als Kartuschenfilter 14a, Sandfilter 14b, oder als hier nicht dargestellter Hohlfaserfilter im Nano- bzw. Ultraporenbereich ausgebildet sein.

Ein Enthärter 15, beispielsweise als Doppelenthärter dargestellt, wird in der Regel mit starksaurem, kationenhaltigem Harz gefüllt, welches bei Erschöpfung regelmäßig mit NaCl-Lösung aus der Salzsoleaufbereitung 16 zu regenerieren ist.

Dabei ist es wichtig, den Füllstand des Salzes im Salzsolebehälter 16 zu überwachen. Dies erfolgt mit einer Wäageeinrichtung 17, die als eigenständiges konstruktives Untergestell ausgebildet ist.

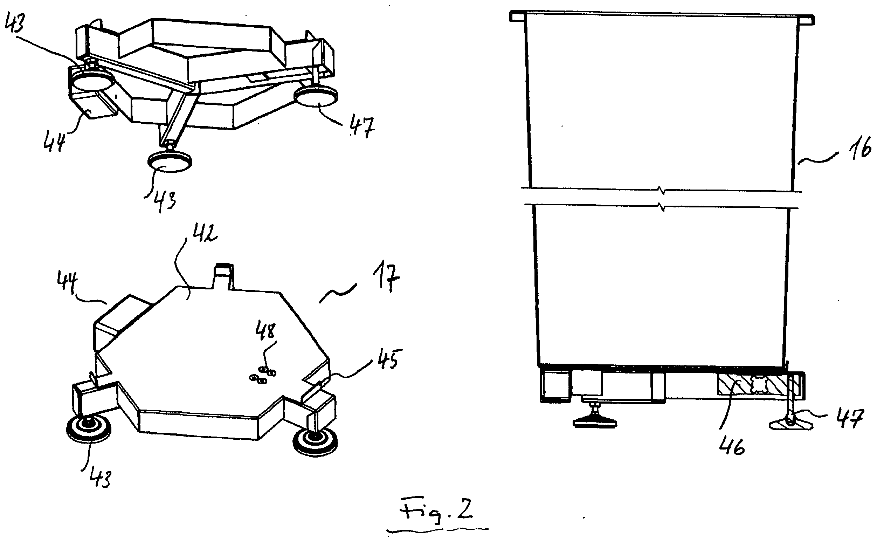

Gemäß Figur 2 besteht die Wäageeinrichtung 17 aus einer Wäagezelle 46, deren Signal von Elektronik 44 an der Wäageplattform 42 verstärkt, elektronisch aufbereitet, oder auch von Elektronik 2 als auch von einer evtl. nachfolgenden Elektronik 5 verarbeitet werden kann. Dabei können voreingestellte Gewichtsgrenzwerte des Solebehälters überwacht u. optisch oder akustisch zur Anzeige bzw. bei EDV technischer Verarbeitung ferndiagnostiziert werden.

Die Wäagezelle 46 ist an der Wäageplattform 42 mittels Schrauben 48 so befestigt, dass ein Drittel des Sole- bzw. Salzgewichtes auf dem Messfuß 47 lastet. Zur Seitenführung des Solebehälters sind die Seitenbegrenzungen 45 angebracht.

Mittels einer Elektrolyseeinrichtung 18 kann während des Regenerationsvorgangs des Enthärters 15 chlorhaltige Lösung aus der zur Elektrolysezelle 18 fließenden Salzsole gebildet werden. Dabei versteht es sich, dass die Chlorkonzentration von der Solekonzentration, aber im Wesentlichen von der Höhe der elektrisch zugeführten Leistung zur Elektrolysezelle abhängt. Der mikrobielle Aufwuchs im Enthärterharz wird dadurch stark reduziert.

19 zeigt eine doppelte Kohlefilter/Dechlorierungseinrichtung, die zur Filtration des Chlors eingesetzt wird.

Eine Filterstufe 20 kann als Feinfilterstufe kleinste Partikel aus dem Filterwasser 7 entfernen, bevor es beispielweise einer Umkehrosmoseanlage oder einer Trinkwasseranlage zugeführt wird.

Die Aktor-Sensor-Einheit 3 kann mit einem elektronischen Wasserzähler 21 zur Registrierung und Meldung der Wasserverbräuche bestückt werden.

Zur Überwachung des Chlorgehalts der zugeführten Flüssigkeit befindet sich in einer Chlorsensorkammer 29 erfindungsgemäß ein Chlorsensor 30, zur Messung des gesamten Chlors .

Die Chlorsensorkammer 29 hat einen Zufluss und einen freien Abfluss. Direkt vor der Sensorkammer befindet sich ein Freigabeventil 28.

Üblicherweise kann die zugeführte Flüssigkeit vom Wasserversorger mit Chlor unterschiedlicher Konzentrationen gechlort werden, wobei je nach Hygienezustand unter Umständen zeitweise kein Chloreintrag vorhanden ist. In diesem Fall ist ohne weitere Maßnahme keine Aussage über die ordnungsgemäße Funktion des Sensors 30 möglich.

Zur regelmäßigen Überprüfung des Chlorsensors werden ein Testventil 27, ein Soleansaugventil 24, und das Freigabeventil 28 geöffnet und die Elektrolysezelle 18 eingeschaltet. Dabei wird Sole beziehungsweise Chlorhaltige Lösung in einem ausgewählten Konzentrationsverhältnis aus dem Solebehälter 16 übet das einstellbare Soleansaugventil 24 und eine Pumpe 23 angesaugt, mit Flüssigkeit über eine Flussdrossel 25 vermischt, zur Messkammer 29 weitergeleitet, über den Chlorsensor 30 registriert und mit der Elektronik 2 beziehungsweise 5 ausgewertet. Durch diesen regelmäßigen Test kann die ordnungsgemäße Funktion der Messzelle 30 sichergestellt werden.

Es liegt im Rahmen der Erfindung, eine Bereitstellung und Überwachung der Natriumchloridsolelösung auch ausschließlich zum Zwecke eines Chlorsensormonitorings, unabhängig von einem Enthärter oder weiteren Filterstufen einzusetzen. Dabei sind die Ansaugleitung der Solelösung und die Elektrolysezelle für die elektrolytische Chlorerzeugung unabhängig von einer Soleansaugleitung und einer Elektrolysezelle des Enthärters ausgeführt.

Die Pumpe 23 ist vorzugsweise als Venturipumpe dargestellt, aber andere Pumpentypen sind ebenfalls zur Erreichung der Funktion möglich, wobei in diesem Falle eine Zudosierung der chlorhaltigen Lösung mittels einer nicht dargestellten Pumpe aus Leitung 24a in Leitung 25a erfolgt.

Zur Überwachung der korrekten Kohlefilterfunktion/Dechloriereinrichtung 19 kann zunächst ein stromaufwärtiges Ventil, beispielsweise 40 oder 27, geöffnet werden.

Ebenso wird das Freigabeventil 28 geöffnet. Falls sich Chlor in der zugeführten Flüssigkeit befindet, wird dies über den vorher verifizierten Chlorsensor 30 registriert. Danach werden nacheinander die Ventile 33 nach der ersten Filterstufe, 31 nach der zweiten Filterstufe oder auch 32 nach einer Feinfilterstufe 20 und das Chlorfreigabeventil 28 geöffnet. So können die Filterstufen des Kohlefilters getestet werden. Registriert der Chlorsensor das Nichtvorhandensein von Chlor, ist die Überprüfung des Filters erfolgreich abgeschlossen. Es liegt im Sinne der Erfindung, dass auch diese Messung eigenständig ausgeführt und EDV technisch registriert werden kann.

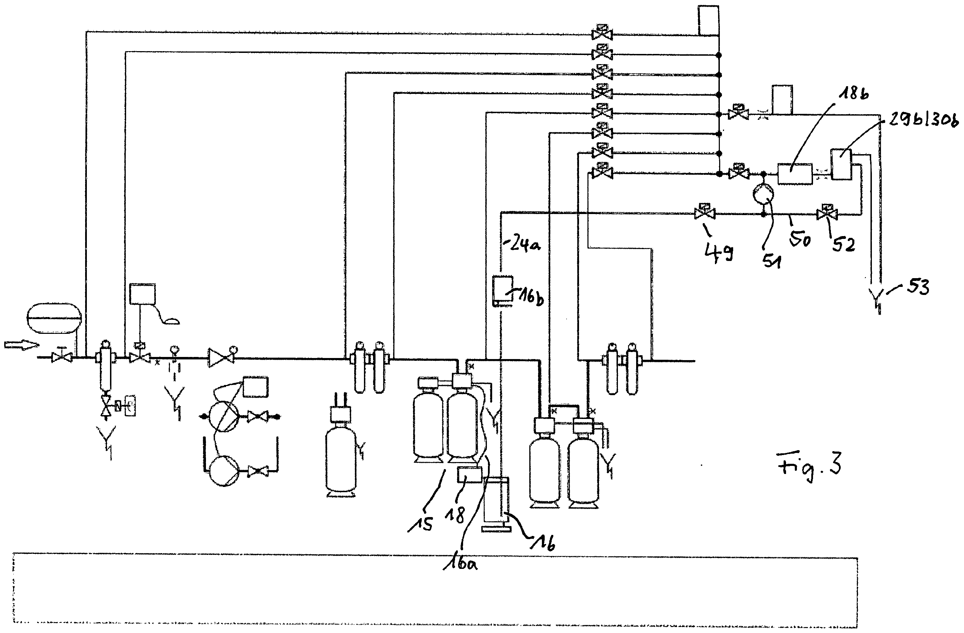

Figur 3 zeigt eine weitere Vorfiltrationseinheit, die sich von derjenigen der Figur 1 durch die Maßnahmen zur Sicherstellung einer ordnungsgemäßen Funktion der Chlormesszelle und zu deren Überprüfung unterscheidet. Dabei wird Sole bzw. chlorhaltige Lösung in einem vorbestimmten Konzentrationsverhältnis entweder aus dem Solebehälter 16, der zur Regenerierung des Enthärters 15 angeordnet ist, oder aus einem getrennt bereit gestellten Solebehälter 16b angesaugt. Die zugehörige Chlortestleitung 24a mündet hinter einem Absperrventil 49 in einen Chlortest-Zirkulationskreis 50 ein, in den im Uhrzeigersinn, in dem die angesaugt Sole zirkuliert, nacheinander eine Pumpe 51 eine Elektrolysezelle 18b und eine Messkammer 29b mit einem Chlorsensor sowie ein weiteres Absperrventil 52 eingebaut sind. Die Chlormesskammer 29b ist mit einem freien Abfluss 53 verbunden, wie dies auch bei der Ausführungsform der Figur 1 der Fall ist. Bei dieser Ausführungsform wird die Sole - anders als bei der Ausführungsform der Figur 1 - direkt aus dem Solebehälter 16 angesaugt, und nicht als chlorhaltige Flüssigkeit aus der Verbindungsleitung 16a zwischen der Elektrolysezelle 18 und dem Enthärter 15 abgezweigt.

Aus dem zur Regenerierung des Enthärters 15 vorgesehenen Solebehälter 16 oder einem optional verfügbaren Solebehälter 16b wird dabei eine geringe Solemenge entnommen und durch den Chlor-Generator 18b und die Chlor-Messzelle 29b zirkuliert. Dies erfolgt erfindungsgemäß in regelmäßigen Zeitabständen, um die Chlormesszelle aktiv zu halten und eine Funktionskontrolle durchzuführen. Dabei soll die Chlor-Messzelle bzw. der Chlorsensor 30 an einer kurzen, immer gleichen Einschaltdauer des Chlorgenerators 18b einen Messwert in einem vorgegebenen Bereich aufzeigen. Nach dieser Aktivierung und Erfassung wird die gesamte Leitung zum Solebehälter hin frei gespült. Das Freispülintervall ist so festgelegt, dass die entnommene Solemenge wieder ergänzt wird.

Es liegt im Rahmen der Erfindung, dass anstelle des Solebehälters 16b ein Behälter mit Chlorbleichlauge oder Chlordioxid oder einer ähnlichen chlorhaltigen Flüssigkeit verwendet wird. In diesem Fall entfällt der Chlorgenerator 18b.

Durch die oben beschriebene Aktivierung der Chlormesszelle wird zuverlässig verhindert, dass diese passiv wird. Dabei werden nur minimale Mengen an NaCl verbraucht.

Abgesehen von den oben beschriebenen Merkmalen zur Aktivierung und Kontrolle der Chlormesszelle stimmt die Vorfiltrationseinheit der Figur 3 mit derjenigen der Figur 1 überein, so dass deren Bezugszeichen auch für die Ausführungsform der Figur 3 zutreffen. Die Anordnung der acht Ventile 37, 38, 39, 40, 27, 33, 31, 32 ist rein schematisch dargestellt. Diese Ventile und ihre zugehörigen Betätigungsglieder, die in der Beschreibung auch als Aktoren bezeichnet sind, können - anders als in den Zeichnungen dargestellt - auch an den Stellen vorgesehen sein, an denen die zugehörigen Stichleitungen von der Wasserleitung 6a abzweigen.

Zur Überwachung der Filterstufen 9, 14, 20 wird ein Druckaufnehmer 41 wahlweise und nacheinander mit den an den Filterstufen herrschenden Drücken über die in Fig. 1 dargestellten Ventile 37, 38, 39, 40, 31, 32 vor oder nach den Filterstufen beaufschlagt.

So findet beispielweise die Überwachung des Druckabfalles der Filterstufe 9 mittels Messung des Eingangsdruckes über das stromaufwärtige Ventil 37 und die Überwachung des Ausgangdruckes durch das nachfolgende Ventil 38 statt.

Äquivalent zu der vorgenannten Messung sind in Figur 1 die Messung der Druckabfälle durch Schaltung der Ventile 39/40 für die Filterstufen 14 und der Ventile 31/32 für Filterstufe 20 nachzuvollziehen.

Die Ermittlung der Druckabfälle an dem Enthärter 15 und der Dechlorierung 19 ist durch ein aufeinanderfolgendes Schalten der Ventile 40, 27, 33, 31 ebenfalls möglich.

Eine atmosphärische Entlastung des Drucksensors 41 generell oder zwischen zwei Messungen kann über Ventil 34 als auch 28 erfolgen.

Durch Messung des Flusses durch die Leitung 6a mit Wasseruhr/Flussmesser 21 oder auch einer entsprechenden Flussmessung in einer nachfolgenden Aufbereitung können die an den Filtern gemessenen Druckwerte mittels Elektronik 2 ,5 auf Standard- bzw. Mittelwerte errechnet und ein Warn-, Austausch-, Spül-, oder Wartungszeitpunkt für voreingestellte Druckdifferenzen prognostiziert werden.

Da es sich bei der Ermittlung der Filter - Druckdifferenzen in der Regel um relative Messungen handelt, ist die Verwendung eines einzigen Drucksensors 41 sowohl kostenmäßig als auch hinsichtlich des Kalibrieraufwandes vorteilhaft.

Normalerweise sind die Wassereingangsdrücke an der Leitung 6a z.B. am Filter 9 bekannt, sodass der Drucksensor 41 vor Beginn eines Messzyklus mit einem bekannten Druck beaufschlagt im Rahmen einer Wartung oder Techniker Inspektion zu verifizieren ist.

Eine vorteilhafte Ausgestaltung der Druckmessung ist die Ermittlung von Druckmittelwerten mittels Elektronik 2, 5 an den jeweiligen Filtern 9, 14, 15, 19, 20 indem beispielsweise 50 Messungen zu einem Mittelwert zusammengefasst und über einen beispielhaften Zeitraum von 1000 Betriebsstunden dargestellt werden . Änderungen, die auf ein Lebensdauerende des Sensors 41 oder des Verblockens der o.a. Filter zurückzuführen sind, lassen sich damit EDV - technisch erkennen bzw. prognostizieren und fernabfragen.

Zur Überwachung der korrekten Funktion des Enthärters 15 wird zunächst das Ventil 40 geöffnet und einem Calciumsensor 36 durch das geöffnete Ventil 34 Hartwasser über eine Messkammer 35 zugeführt.

Im Anschluss wird enthärtete Flüssigkeit über die Flussdrossel 25 und Ventile 27, 34 in die Messkammer 35 zum lonensensitiven Calciumsensor 36 geleitet.

**Leqende**

| | |
|---|---|
| 1. | Vorfiltration mit Sensorpaket |
| 2. | Elektronik Sensorpaket |
| 3. | Aktor- und Sensoreinheit |
| 4. | Vorfiltrationskomponenten |
| 5. | Elektronik Nachfiltration |
| 6. | Wassereingang |
| 7 | Filterwasser |
| 8. | Absperrventil |
| 9. | Rückspülbarer Vorfilter mit Reinigungsventil |
| 10. | Sicherheitsabspertventil |
| 11 . | Rohrtrenner |
| 12. | Rückflussverhinderer |
| 13. | Druckerhöhungseinheit |
| 14. | Feinfilterstufe 2 |
| 15. | Enthärtungsstufe |
| 16. | Salzsoleaufbereitung/ Soletank |
| 1 7. | Wäageeinheit |
| 18. | Elektrolysezelle |
| 1 9. | Dechlorierungsstufe/ Kohlefilter |
| 20. | Feinfilterstufe 3 |
| 21. | Wasseruhr/ Flussmesser |
| 22. | Leckagemeldung mit Sensor |
| 23. | Solepumpe |
| 24. | Soleansaugventil |
| 25. | Flussdrossel |
| 26. | Rückflussverhinderung |
| 27. | Chlorsensortestventil / Calciumprüfventil I |
| 28. | Chlorsensorfreigabeventil |
| 29. | Chlorsensorkammer |
| 30. | Chlorsensor |
| 31 . | Chlorprüfventil II / Feinfilterstufe 3 Eingangsdruck |

| | |
|---|---|
| 32. | Chtorprüfventil III / Feinfllterstufe 3 Ausgangsdruck |
| 33. | Chlorprüfventil I |
| 34. | Calciumsensorfreigabeventil |
| 35. | Calciumsensorkammer |
| 36. | Calciumsensor |
| 37. | Feinfilterstufe 1 Eingangsdruck |
| 38. | Feinfilterstufe 1 Ausgangsdruck |
| 39. | Feinfilterstufe 2 Eingangsdruck |
| 40. | Feinfilterstufe 2 Ausgangsdruck/ Calciumtestventil |
| 41 | Drucksensor |
| 6a | Leitungen |
| 16a | |
| 19a | |
| 24a | |
| 25a | |
| 42. | Plattform |
| 43. | Einstellbare Füße |
| 44. | Elektronik |
| 45. | Seitenbegrenzung |
| 46. | Wäagezelle |
| 47. | Messfuss |
| 48. | Befestigung Wäagezelle |
| 49. | Absperrventil |
| 50. | Chlortest-Zirkulationskreis |
| 51 | Pumpe |
| 52. | Absperrventil |
| 53. | freier Abfluss |

## Patentansprüche

1. Wasseraufbereitungsanlage, insbesondere Vorfiltrationseinheit der Wasseraufbereitungsanlage, mit einer Chlorsensoreinrichtung und einer damit verbundenen Auswertelektronik,
**dadurch gekennzeichnet,**
**dass** die Wasseraufbereitungsanlage einen Salzsoletank (16,16b) enthält, der über eine Chlortestleitung (24a, 50) mit der Chlorsensoreinrichtung (29, 30; 29b, 30b) verbunden ist, wobei in die Chlortestleitung eine Elektrolysezelle (18, 18b), eine Pumpe (23, 51) sowie ein Freigabeventil (28, 49) eingeschaltet sind und die Chlorsensoreinrichtung (29,30,29b,30b) einen online-messenden Gesamtchlorsensor umfasst, wobei die Wasseraufbereitungsanlage dazu ausgelegt ist, dem online-messenden Gesamtchlorsensor regelmäßig elektrolytisch erzeugtes Chlor bekannter Konzentration zuzuführen, diese über den Gesamtchlorsensor zu registrieren und mit der Auswerteelektronik auszuwerten, um die Funktion des online-messenden Gesamtchlorsensors regelmäßig zu überprüfen, um zu verhindern, dass die Chlorsensoreinrichtung passiv wird und um einem Anwender zu ermöglichen, die Funktionalität der Wasseraufbereitungsanlage per online-Zugriff zu bewerten und auf dieser Basis eine Ferndiagnose über den aktuellen Betriebszustand zu erhalten.

2. Wasseraufbereitungsanlage, insbesondere Vorfiltrationseinheit der Wasseraufbereitungsanlage, mit einer Chlorsensoreinrichtung und einer damit verbundenen Auswertelektronik,
**dadurch gekennzeichnet,**
**dass** die Wasseraufbereitungsanlage einen Behälter mit einer chlorhaltigen Lösung von einem ausgewählten Konzentrationsverhältnis, vorzugsweise Chlorbleichlauge oder Chlordioxid, enthält, der über eine Chlortestleitung (24a, 50) mit der Chlorsensoreinrichtung (29, 30; 29b, 30b) verbunden ist, wobei in die Chlortestleitung eine Pumpe (23, 51) sowie ein Freigabeventil (28, 49) eingeschaltet sind und die Chlorsensoreinrichtung (29,30,29b,30b) einen online-messenden Gesamtchlorsensor umfasst, wobei die Wasseraufbereitungsanlage dazu ausgelegt ist, dem online-messenden Gesamtchlorsensor regelmäßig Chlor bekannter Konzentration zuzuführen, diese über den Gesamtchlorsensor zu registrieren und mit der Auswerteelektronik auszuwerten, um die Funktion des online-messenden Gesamtchlorsensors regelmäßig zu überwachen, um zu verhindern, dass die Chlorsensoreinrichtung passiv wird und um einem Anwender zu ermöglichen, die Funktionalität der Wasseraufbereitungsanlage per online-Zugriff zu bewerten und auf dieser Basis eine Ferndiagnose über den aktuellen Betriebszustand zu erhalten.

3. Wasseraufbereitungsanlage nach Anspruch 1,
ferner **gekennzeichnet dadurch,**
**dass** die Pumpe (51), die Elektrolysezelle (18b) und die Chlorsensoreinrichtung (29b, 30b) in einem mit der Chlortestleitung (24a) verbundenen Rezirkulationskreis (50) angeordnet sind, in dem die zugeführte Salzsole durch die Elektrolysezelle (18b) und die Chlorsensoreinrichtung (29b, 30b) zirkuliert.

4. Wasseraufbereitungsanlage nach Anspruch 2, ferner **gekennzeichnet dadurch, dass** die Pumpe (51) und die Chlorsensoreinrichtung (29b, 30b) in einem mit der Chlortestleitung (24a) verbundenen Rezirkulationskreis (50) angeordnet sind, in dem die zugeführte chlorhaltige Flüssigkeit durch die Chlorsensoreinrichtung (29b, 30b) zirkuliert.

5. Wasseraufbereitungsanlage nach Anspruch 1 oder 3, ferner **gekennzeichnet durch** eine Enthärtereinrichtung (15), mit der der Salzsoletank (16) über eine Salzsoleleitung (16a) verbunden ist, wobei die Elektrolysezelle (18) in die Salzsoleleitung (16a) eingeschaltet ist, und wobei zwischen der Elektrolysezelle (18) und der Enthärtereinrichtung (15) eine Chlortestleitung (24a) von der Salzsoleleitung (16a) abzweigt, die zu der Chlorsensoreinrichtung (29, 30) führt.

6. Wasseraufbereitungsanlage nach Anspruch 1,
**dadurch gekennzeichnet,**
**dass** die Pumpe (23) eine Venturipumpe ist und
**dass** zu der Pumpe (23) außerdem eine Wasserleitung (25a) führt, in die eine einstellbare Drossel (25) eingeschaltet ist.

7. Wasseraufbereitungsanlage nach Anspruch 5 oder 6,
**dadurch gekennzeichnet,**
**dass** die Zudosierung der chlorhaltigen Lösung mittels einer weiteren Pumpe (24) aus der Leitung (24a) in die Chlortestleitung (25a) erfolgt.

8. Wasseraufbereitungsanlage nach Anspruch 1,
**dadurch gekennzeichnet,**
**dass** der Salzsoletank (16) auf einer Wägeeinrichtung (17) angeordnet ist.

9. Wasseraufbereitungsanlage nach den Ansprüchen 1 bis 9,
**dadurch gekennzeichnet,**
**dass** eine Dechlorierungseinrichtung (19) in die Wasserleitung (6a), eingeschaltet ist, deren Ausgänge über Leitungen (19a, 6b) und Schaltventile (31,33) mit der Chlorsensoreinrichtung (29) verbunden sind.

## Claims

1. Water treatment system, in particular prefiltration unit of the water treatment system, comprising a chlorine sensor device and an evaluation electronics,
**characterized in that**
the water treatment system contains a brine tank (16, 16b) which is connected to the chlorine sensor device (29, 30; 29b, 30b) via a chlorine test line (24a, 50), wherein in the chlorine test line an electrolysis cell (18, 18b), a pump (23, 51) as well as a release valve (28, 49) are disposed and the chlorine sensor device (29, 30, 29b, 30b) comprises an online measuring overall chlorine sensor, wherein the water treatment system is configured to supply electrolytically generated chlorine of known concentration to the online measuring chlorine sensor at regular intervals, to register this concentration via the overall chlorine sensor and evaluate it with the evaluation electronics, in order to check the function of the online measuring overall chlorine sensor at regular intervals, in order to prevent the chlorine sensor device from becoming passive and in order to allow a user to assess the functioning of the water treatment system via online access and to obtain a remote diagnosis on the current operating state on this basis.

2. Water treatment system, in particular prefiltration unit of the water treatment system, comprising a chlorine sensor device and an evaluation electronics,
**characterized in that**
the water treatment system contains a container with a chlorine-containing solution of selected concentration ratio, preferably chlorine bleaching lye or chlorine dioxide, which is connected to the chlorine sensor device (29, 30; 29b, 30b) via a chlorine test line (24a, 50), wherein in the chlorine test line a pump (23, 51) and a release valve (28, 49) are disposed and the chlorine sensor device (29, 30, 29b, 30b) comprises an online measuring overall chlorine sensor, wherein the water treatment system is configured to supply electrolytically generated chlorine of known concentration to the online measuring chlorine sensor at regular intervals, to register this concentration via the overall chlorine sensor and evaluate it with the evaluation electronics, in order to check the function of the online measuring overall chlorine sensor at regular intervals, in order to prevent the chlorine sensor device from becoming passive and in order to allow a user to assess the functioning of the water treatment system via online access and to obtain a remote diagnosis on the current operating state on this basis.

3. Water treatment system according to claim 1,
further **characterized in that**
the pump (51), the electrolysis cell (18b) and the chlorine sensor device (29b, 30b) are arranged in a recirculation circuit (50) connected to the chlorine test line (24a), in which the supplied brine circulates through the electrolysis cell (18b) and the chlorine sensor device (29b, 30b).

4. Water treatment system according to claim 2, further **characterized in that** the pump (51) and the chlorine sensor device (29b, 30b) are arranged in a recirculation circuit (50) connected to the chlorine test line (24a), in which the supplied chlorine-containing liquid circulates through the chlorine sensor device (29b, 30b).

5. Water treatment system according to claim 1 or 3, further **characterized by** a softener device (15), to which the brine container (16) is connected via a brine line (16a), wherein the electrolysis cell (18) is connected in the brine line (16a) and wherein a chlorine test line (24a) branches off from the brine line (16a) between the electrolysis cell (18) and the softener device (15), which line leads to the chlorine sensor device (29, 30).

6. Water treatment system according to claim 1,
**characterized in that**
the pump (23) is a venturi pump, and
a water line (25a) leads to the pump (23), into which line an adjustable throttle (25) is connected.

7. Water treatment system according to claim 5 or 6,
**characterized in that**
the dosing of chlorine-containing solution is effected by means of a further pump (24) from the line (24a) into the chlorine test line (25a).

8. Water treatment system according to claim 1,
**characterized in that**
the brine tank (16) is arranged on a weighing device (17).

9. Water treatment system according to claims 1 to 9,
**characterized in that**
a dichlorination device (19) is connected in the water line (6a), the outlets of which are connected to the chlorine sensor device (29) via lines (19a, 6b) and switch valves (31, 33).

## Revendications

1. Installation de traitement de l'eau, en particulier unité de préfiltrage de l'installation de traitement de l'eau, avec un système de détection du chlore et un dispositif électronique d'évaluation raccordé à celui-ci,
**caractérisée en ce que**
l'installation de traitement de l'eau contient une cuve de saumure (16, 16b), qui est raccordée au système de détection du chlore (29, 30 ; 29b, 30b) par l'intermédiaire d'une conduite de test du chlore (24a, 50), dans laquelle une cellule d'électrolyse (18, 18b), une pompe (23, 51) ainsi qu'une valve de libération (28, 49) sont activées dans la conduite de test du chlore et le système de détection du chlore (29, 30, 29b, 30b) comprend un capteur de chlore total à mesure en ligne, dans laquelle l'installation de traitement de l'eau est conçue pour amener régulièrement au capteur de chlore total à mesure en ligne du chlore produit par électrolyse présentant une concentration connue, pour enregistrer celle-ci par l'intermédiaire du capteur de chlore total et l'analyser avec le dispositif électronique d'analyse pour vérifier régulièrement le fonctionnement du capteur de chlore total à mesure en ligne pour empêcher que le système de détection du chlore ne soit passif et pour permettre à un utilisateur d'apprécier la fonctionnalité de l'installation de traitement de l'eau par un accès en ligne et pour obtenir, sur cette base, un diagnostic à distance sur l'état de fonctionnement du moment.

2. Installation de traitement de l'eau, en particulier unité de préfiltrage de l'installation de traitement de l'eau, avec un système de détection du chlore et un dispositif électronique d'évaluation raccordé à celui-ci,
**caractérisée en ce que**
l'installation de traitement de l'eau contient un contenant avec une solution contenant du chlore présentant un rapport de concentration choisi, de préférence de l'hypochlorite de sodium ou du dioxyde de chlore, qui est raccordé au système de détection du chlore (29, 30 ; 29b, 30b) par l'intermédiaire d'une conduite de test du chlore (24a, 50), dans laquelle une pompe (23, 51) ainsi qu'une soupape de libération (28, 49) sont activées dans la conduite de test du chlore et le système de détection du chlore (29, 30, 29b, 30b) comprend un capteur de chlore total à mesure en ligne, dans laquelle l'installation de traitement de l'eau est conçue pour amener régulièrement au capteur de chlore total à mesure en ligne du chlore présentant une concentration connue, pour enregistrer celle-ci par l'intermédiaire du capteur de chlore total et l'analyser avec le dispositif électronique d'analyse, pour surveiller régulièrement le fonctionnement du capteur de chlore total à mesure en ligne pour empêcher que le système de détection du chlore en soit passif et pour permettre à un utilisateur d'apprécier la fonctionnalité de l'installation de traitement de l'eau par un accès en ligne et pour obtenir sur cette base un diagnostic à distance sur l'état de fonctionnement du moment.

3. Installation de traitement de l'eau selon la revendication 1,
**caractérisée en outre en ce**
**que** la pompe (51), la cellule d'électrolyse (18b) et le système de détection du chlore (29b, 30b) sont disposés dans un circuit de recirculation (50) raccordé à la conduite de test du chlore (24a), dans lequel la saumure amenée circule à travers la cellule d'électrolyse (18b) et le système de détection du chlore (29b, 30b).

4. Installation de traitement de l'eau selon la revendication 2, **caractérisée en outre en ce que** la pompe (51) et le système de détection du chlore (29b, 30b) sont disposés dans un circuit de recirculation (50) raccordé à la conduite de test du chlore (24a), dans lequel le liquide contenant du chlore amené circule à travers le système de détection du chlore (29b, 30b).

5. Installation de traitement de l'eau selon la revendication 1 ou 3, **caractérisée en outre par** un système adoucisseur (15), auquel la cuve de saumure (16) est raccordée par l'intermédiaire d'une conduite de saumure (16a), dans laquelle la cellule d'électrolyse (18) est activée dans la conduite de saumure (16a), et dans laquelle une conduite de test du chlore (24a) forme un embranchement, entre la cellule d'électrolyse (18) et le système adoucisseur (15), avec la conduite de saumure (16a), qui mène au système de détection du chlore (29, 30).

6. Installation de traitement de l'eau selon la revendication 1,
**caractérisée en ce**
**que** la pompe (23) est une pompe à effet Venturi, et
**que** mène par ailleurs à la pompe (23) une conduite d'eau (25a), dans laquelle un étrangleur (25) réglable est activé.

7. Installation de traitement de l'eau selon la revendication 5 ou 6,
**caractérisée en ce**
**que** le dosage de la solution contenant du chlore est effectué depuis la conduite (24a) dans la conduite de test du chlore (25a) au moyen d'une autre pompe (24).

8. Installation de traitement de l'eau selon la revendication 1,
**caractérisée en ce**
**que** la cuve de saumure (16) est disposée sur un système de pesage (17).

9. Installation de traitement de l'eau selon les revendications 1 à 9,
**caractérisée en ce**
**qu'**un système de déchloration (19) est activé dans la conduite d'eau (6a), dont les sorties sont raccordées au système de détection du chlore (29) par l'intermédiaire de conduites (19a, 6b) et de valves de commutation (31, 33).
